# EUROPÄISCHE PATENTSCHRIFT

(11) **EP 0 624 349 B1**
(45) Veröffentlichungstag und Bekanntmachung des Hinweises auf die Patenterteilung: **29.01.1997**
(21) Anmeldenummer: 94107029.4
(22) Anmeldetag: 05.05.1994
(51) Int. Cl.: A61B 17/58, A61B 17/24, A61B 17/12

(54) **Vorrichtung zur Tamponade und zum Offenhalten von knochenbegrenzten Körperhöhlen**
Device for tamponade in and maintenance of bony body cavities
Dispositif de tamponnement et de maintenir des cavités osseuses corporelles

(30) Priorität: 12.05.1993 DE 4315821
(43) Veröffentlichungstag der Anmeldung: 17.11.1994
(73) Patentinhaber: Milewski, Christian, Dr. med., D-97209 Veitshöchheim (DE)
(72) Erfinder: Milewski, Christian, Dr. med., D-97209 Veitshöchheim (DE)
(74) Vertreter: Fuchs, Luderschmidt & Partner Patentanwälte

(56) Entgegenhaltungen:
- EP-A- 0 057 597
- EP-A- 0 141 589
- AT-A- 384 945
- DE-A- 3 736 604
- US-A- 3 800 788

## Beschreibung

Die Erfindung betrifft eine Vorrichtung zur Tamponade und zum Offenhalten von knochenbegrenzten Körperhöhlen und Gängen nach chirurgischer Manipulation nach dem Oberbegriff des Patentanspruchs 1.

Es ist bereits ein als Ballon ausgebildeter Katheter der gattungsgemäßen Art bekannt, der zum Erhalt und zur Wiederherstellung begrenzender Knochenwände einer Körperhöhle nach einem chirurgischen Eingriff die Form eines Tetraeders aufweist (DE 3736604 A1). Ein solcher Ballonkatheter dient zur Rückhaltung verletzter Knochenwände in ihrer anatomisch richtigen Lage und insbesondere zum Vermeiden einer Durchbiegung der frakturierten Augenhöhle bei chirurgischen Eingriffen. Der aus DE 37 36 604 A1 bekannte Ballonkatheter weist ein schlauchförmiges Anschlußteil auf, das an eine Spritze angeschlossen werden kam. Während eine der Flächen des Tetraeder-Ballons im wesentlichen unelastisch ist, sind die übrigen Flächen elastisch ausgebildet, so daß sich diese beim Befällen des Ballons halbkugelförmig verformen.

Neben der Behandlung von Frakturen besteht jedoch auch ein Bedarf an einem geeigneten Katheter für die Heilbehandlung nach chirurgischen Eingriffen in Körperhöhlungen, insbesondere im Bereich der Siebbein- und Stirnhöhlenchirurgie, der Traumatologie der Schädelbasis, der Tumorchirurgie und der Rekonstruktion von flachen Schädelhöhlen. Dazu ist der bekannte, als tetraederförmige Ballon ausgebildete Katheter nicht geeignet.

So werden z.B. bei der Chirurgie des Siebbeins, d.h. der zwischen der Nase und der Augenhöhle gelegenen Region, vor allem gutartige Tumoren (Polypen) und eine chronisch verschwollende Schleimhaut operiert. Nach dem Entfernen des veränderten Gewebes kann es zu heftigen Blutungen und Narbenbildung kommen. Um Blutung und Narben zu verhindern, wird üblicherweise eine Salbenstreifentamponade in die flachen Siebbeinhöhlen eingelegt. Diese haben jedoch die Neigung, bakteriell zu verkeimen. Zusätzlich wächst Schleimhaut in die Tamponade. Nach spätestens 2 Tagen müssen deshalb diese Salbenstreifen entfernt werden, was mit erneuter Blutung und Schmerzen für den Patienten verbunden ist. Außerdem wird die beginnende Heilung durch Überwachsen der freiliegenden Knochenwände durch Mukosa wieder gestört.

Bei der Chirurgie im Bereich der Stirnhöhle stellt die Eigenart der Stirnhöhle selbst das größte Problem dar. Die teilweise sehr flache Stirnhöhle hat ihre Belüftungswege am Boden über das Siebbein in die Nase. Beim Operieren müssen diese Belüftungswege unbedingt offen gehalten werden. Bei Stirnhohlenvorderwandbrüchen und chronischen Entzündungen kommt es zum Verschluß der Belüftungswege und damit zu gefährlichen Entzündungen und Sekretstau. Da die Belüftungswege sehr eng sind, neigen sie stark zur narbigen Verengung. Ein wirksames Mittel dagegen ist bisher nicht vorhanden, da eine Tamponade aus Salbenstreifen wegen der viel zu kurzen Liegedauer nutzlos ist.

Die Schädeldecke kann bei Verkehrsunfällen und Tumoren so stark geschädigt sein, daß Hirnwasser über die Nase ausfließt. Ein offener Zugang zum Schädelinneren birgt dann die Gefahr einer aufsteigenden Infektion. Üblicherweise wird das Nasendach operativ freigelegt und der Bruch mit geeignetem Material wasserdicht verschlossen. Dazu wird vorzugsweise Spenderhirnhaut vom Menschen verwendet, die mit Fibrinkleber am Knochen angeklebt wird. Diese Klebung allein genügt jedoch nicht, vielmehr muß zusätzlich eine Tamponade erfolgen, die wiederum die oben erwähnten Nachteile mit sich bringt.

In der Tumorchirurgie der Nasenhöhle schließlich ist vor allem das Wiederüberziehen der freiliegenden Knochenteile mit der Schleimhaut bei der gebildeten großen Operationshöhle von Bedeutung. Außerdem muß ein Nachbluten zuverlässig verhindert werden.

Der Erfindung liegt die Aufgabe zugrunde, eine Vorrichtung für die Heilbehandlung von Körperhöhlungen zu schaffen, durch die bei der Siebbein- und Stirnhöhlenchirurgie der nachoperative Heilverlauf günstig beeinflußt werden kann und die mit Salbenstreifentamponaden verbundenen Nachteile vermieden werden.

Die Lösung der Aufgabe erfolgt durch die Merkmale des Patentanspruchs 1. Vorteilhafte Ausgestaltungen sind Gegenstand der Ansprüche 2 bis 10.

Die erfindungsgemäße Vorrichtung ist aufgrund ihrer der jeweiligen Körperhöhle angepaßten Form in der Lage, die Höhlung vollständig auszufüllen und ggf. flüssigkeitsdicht abzuschließen. Das konisch geformte Anschlußteil des Ballons hält dabei eine ausreichend große Passage offen, durch die sich der Ballon wieder leicht entfernen läßt.

In der Siebbeinchirurgie ergibt sich mit der erfindungsgemäßen Vorrichtung eine deutliche Verbesserung, da zuverlässige Blutstillung und eine Verhinderung von Narben erreicht werden können, wozu auch das gewebefreundliche Silikon gemäß Anspruch 2 beiträgt, das nicht verkeimt und die Reepithelisierung der Knochenwände beschleunigt.

Bei der Stirnhölenchirurgie bietet der Ballon dagegen durch die lange Liegemöglichkeit und die Gewebeverträglichkeit die Möglichkeit, den Ausführungsgang der Stirnhöhle mehrere Wochen lang offen zu halten, eine Zeit die ausreicht, um ein Überwachsen der Wände mit Schleimhaut zu erreichen, so daß eine Wiederverengung praktisch nicht mehr vorkommt. Gleichzeitig kann bei Trümmerbrüchen der Vorderwand der Ballon die wiedereingesetzten Bruchstücke in ihrer Lage halten, so daß ein Einheilen in Normalposition möglich wird. Hierzu kann ggf. die undehnbare Stützfläche gemäß Anspruch 3 und/oder 4 zusätzlich beitragen.

Die zuletzt genannten Vorteile kommen auch bei der Rekonstruktion der Schädelbasis, wenn Hirnwasser infolge Hirnhautzerreißens austritt, zur Geltung. Der Ballon preßt die Dura so fest an die Knochenbruchstücke, daß eine Abdichtung des Gehirnwassers auch bei nicht exaktem Kleben möglich ist. Durch das exakte Anlegen der gewebeverstärkten Stützfläche an die Schädelbasis erfolgt eine Abstützung in regelrechter Lage bei Rekonstruktion der frakturierten Rhinobasis. Der Ballon muß erst entfernt werden, wenn die Einheilung der Dura schon eingesetzt hat. Die Entfernung des Ballons verursacht keine neuerlichen Schädigungen und Schmerzen.

In der Tumorchirurgie stellt der Ballon eine gewebeverträgliche Tamponademöglichkeit der Operationshöhle dar, die sich optimal den anatomischen Gegebenheiten anpaßt, das Infektionsrisiko der Tamponade ausschließt, eine lange Liegedauer aufweist, die ein Wiederüberziehen des Knochens mit Schleimhaut zuläßt und die Nachblutungsneigung sicher kontrolliert.

Weitere Merkmale und Vorteile der Erfindung ergeben sich aus der nachfolgenden Beschreibung eines Ausführungsbeispiels anhand der Zeichnung. In dieser zeigen:
Fig. 1a, 1b und 1c Ansichten eines erfindungsgemäßen Ballonkatheters von oben, von der Längsseite und von der Stirnseite,
Fig. 2 ein Ventilstück für den Ballonkatheter,
Fig. 3 einen schematischen Längsschnitt durch einen menschlichen Schädel mit einem in das Siebbein eingeführten Ballonkatheter,
Fig. 4 einen schematischen Längsschnitt durch einen menschlichen Schädel mit einem in die Stirnhöhle eingeführten Ballonkatheter.

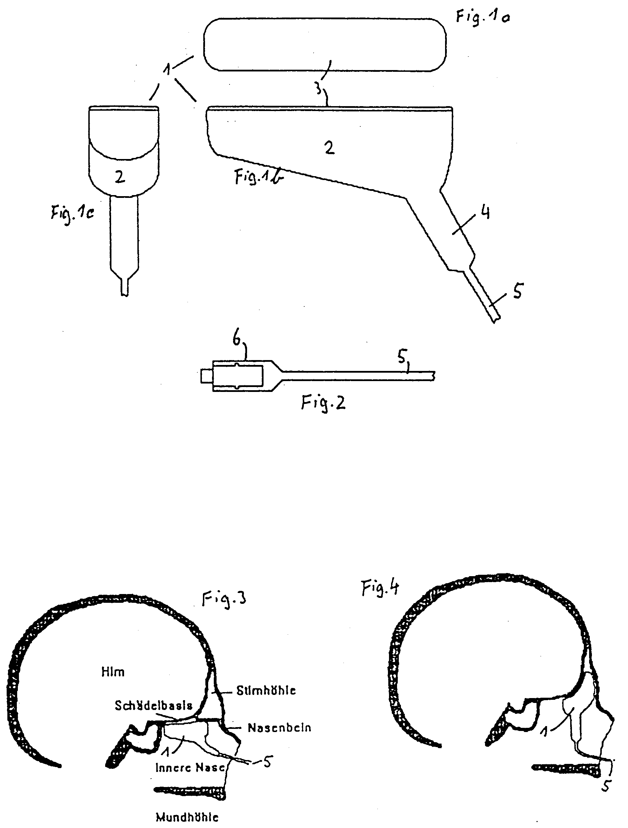

Der in Fig. 1a bis 1c schematisch dargestellte Katheter hat die Form eines langgestreckten keilförmigen, im Querschnitt halbrunden Ballons 1 mit einer flachen Seite 3. Der runde Teil 2 des Ballons besteht aus einem dehnbaren Silikonelastomer und ist in seiner Form an die Körperhöhlung angepaßt, in die der Katheter eingeführt werden soll. Individuelle Abweichungen werden durch die elastische Dehnbarkeit des Silikonmaterials ausgeglichen.

Die flache Seite 3 besteht ebenfalls aus Silikonelastomer, in das jedoch eine Lage aus einem Dracon-Gewebe eingelegt ist, so daß die flache Seite 3 sich nicht elastisch ausdehnen kann. Bei entsprechender Applizierung kann sich der Ballonkatheter mit diesem Teil an einer glatten Fläche abstützen oder an daran anlegen, um diese zu verschließen oder zu rekonstruieren.

An einem Ende des halbrunden, elastischen Ballonteils 2 ist ein Anschlußteil 4 ausgebildet, das mit dem Inneren des Ballons in Verbindung steht. Das Anschlußteil 4 dient zum Anschluß einer flexiblen Zuleitung 5 mit einem darin vorgesehenen Ventilstück 6, ferner zum Offenhalten eines genügend großen Passageweges für die Entfernung. Es ist zu diesem Zweck konisch geformt und besteht aus verstärktem Material. Die Längsachse des Anschlußteils 4 ist gegenüber der flachen Seite 3 um etwa 60° angewinkelt, damit der Katheter gemäß Fig. 3 und 4 optimal in der Nase plaziert werden kann.

Die flexible Zuleitung 5 wird von einem dünnen Schlauch gebildet, der mit einem Faden abgebunden werden kann. Damit ist es möglich, u.U. auf ein Ventil zu verzichten und die Zuleitung im Inneren der Nase unterzubringen, so daß sie von außen nicht sichtbar ist.

Bei der Anwendung wird der Ballonkatheter 1 durch eine Öffnung, die ggfs. operativ geschaffen werden muß, in die zu behandelnde Körperhöhle eingeführt und positioniert. Sodann wird durch die Zuleitung 5 und über das Ventilstück 6 ein Gemisch aus einer isotonischen Kochsalzlösung und einem Röntgenkontrastmittel in den Ballon geleitet. Dies kann z.B. mit Hilfe einer Injektionsspritze erfolgen, wobei zweckmäßigerweise der Innendruck durch ein geeignetes Instrument überwacht wird.

Fig. 3 zeigt einen in die Kieferhöhle eingesetzten Ballon zur Stützung der Schädelbasis nach einer Operation. In Fig. 4 ist ein in die operierte Stirnhöhle zur Tamponade eingesetzter Ballon schematisch dargestellt. Wie ersichtlich, ist der Ballonkatheter an die zu behandelnde Körperhöhle anatomisch angepaßt und kann diese ggfs. vollständig verschließen, so daß keine Gefahr einer aufsteigenden Infektion besteht. Wegen der Gewebeverträglichkeit des Silikonelastomers kann der Ballonkatheter längere Zeit, im Bedarfsfall mehrere Wochen, in der Körperhöhle verbleiben, bis die Ausheilung abgeschlossen ist.

## Patentansprüche

1. Vorrichtung (10) zur Tamponade und zum Offenhalten von knochenbegrenzten Körperhöhlen und Gängen nach chirurgischer Manipulation, mit einem Ballon (1) aus dehnbarem Material, der in die Körperhöhle einführbar und von außerhalb mit einem Fluid fällbar ist und mit einer aus der Körperhöhle herausführbaren Zuleitung (5) für das Fluid, die an einem Ende mit dem Ballon (1) verbunden und am anderen Ende an einen Fluidspeicher anschließbar ist, wobei die Außenkontur des Ballons (1) im mit Fluid gefüllten Zustand der Innenkontur der Körperhöhle angepaßt ist, dadurch gekennzeichnet, daß der Ballon (1) als anatomisch idealisiert geformter Katheter ausgebildet ist, wobei der Ballon (1) zur Anpassung an die menschliche Stirnhöhle oder Siebbeinhöhle keilförmig ausgebildet ist und ein konisch geformtes Anschlußteil (4) aufweist, das eine ausreichend große Öffnung zum Entfernen des Ballons offenhält.

2. Vorrichtung nach Anspruch 1, dadurch gekennzeichnet, daß der Ballon (1) aus einem dehnbaren Silikonelastomer besteht.

3. Vorrichtung nach Anspruch 1 oder 2, dadurch gekennzeichnet, daß der Ballon (1) eine Fläche (3) aufweist, die bei Zugbelastung im wesentlichen undehnbar, jedoch biegeschlaff ausgebildet ist.

4. Vorrichtung nach Anspruch 3, dadurch gekennzeichnet, daß die undehnbare Fläche (3) aus einem Silikonelastomer besteht, in das eine Einlage aus einem undehnbaren Fasergewebe eingebettet ist.

5. Vorrichtung nach Anspruch 4, dadurch gekennzeichnet, daß die Einlage aus einem Polyester besteht.

6. Vorrichtung nach Anspruch 4, dadurch gekennzeichnet, daß die Einlage aus einem Dacron-verstärkten Gewebe besteht.

7. Vorrichtung nach einem der Ansprüche 1 bis 6, dadurch gekennzeichnet, daß das Anschlußteil (4) in einem Winkel von etwa 60° zu der undehnbaren Fläche (3) verläuft.

8. Vorrichtung nach einem der Ansprüche 3 bis 7, dadurch gekennzeichnet, daß die undehnbare Fläche (3) in Anpassung an die längsovale Schädelbasis als längsovale Stützfläche ausgebildet ist.

9. Vorrichtung nach einem der Ansprüche 1 bis 8, dadurch gekennzeichnet, daß der Ballon (1) mit einer dünnen Zuleitung zum Aufblasen und Entlüften verbunden ist.

10. Vorrichtung nach Anspruch 9, dadurch gekennzeichnet, daß das Anschlußteil (4) aus verstärktem Material besteht.

## Claims

1. A device (10) to tampon bone-enclosed body cavities and ducts and to maintain them accessible after surgical manipulation, said device comprising a balloon (1) which is of elastic material and can be introduced into the body cavity and filled with the fluid from the outside, and further comprising a fluid supply means (5) which can be conducted out of the body cavity, said supply means being connected at its one end with the balloon (1) and being connectable at its other end with a fluid reservoir, wherein, when the balloon (1) is filled with fluid, the outer contour thereof is adapted to the inner contour of the body cavity, said device being characterized in that the balloon (1) is designed so as to be a catheter showing an anatomically idealized form, wherein said balloon (1) has a cuneiform shape in order to be adapted to the human frontal sinus or ethmoid sinus and comprises a conical connector (4) which maintains a sufficiently broad orifice open so that the balloon can be removed.

2. The device of claim 1, characterized in that the balloon (1) is made of an extendable silicon elastomer.

3. The device according to claim 1 or 2, characterized in that the balloon (1) which, under tensile load, is developed so as to be stretchless but non-resistant to bending.

4. The device according to claim 3, characterized in that the stretchless surface (3) is made of a silicon elastomer, in which there is embedded an insert of a stretchless fiber tissue.

5. The device of claim 4, characterized in that the insert is made of a polyester.

6. The device of claim 4, characterized in that the insert is made of a Dacron-reinforced tissue.

7. The device of one of claims 1 to 6, characterized in that the connector (4) runs with an angle of about 60° with respect to the stretchless surface (3).

8. The device of one of claims 3 to 7, characterized in that, by virtue of its adaptation to the lengthwise oval skull-base, the stretchless surface (3) is developed as a lengthwise oval supporting surface.

9. The device of one of claims 1 to 8, characterized in that the balloon (1) is connected with a thin feeding pipe for inflation and evacuation of air.

10. The device of claim 9, characterized in that the connector (4) is made of reinforced material.

## Revendications

1. Dispositif pour tamponner des cavités corporelles et des canaux et pour les maintenir ouverts après une manipulation chirurgicale, ledit dispositif comportant un ballon (1) en matière élastique que l'on peut introduire dans la cavité corporelle et remplir d'un fluide de l'extérieur, et comportant de plus un conduit d'amenée (5) pour le fluide que l'on peut conduire de la cavité corporelle, ledit conduit d'amenée étant lié avec l'un des ses bouts au ballon (1) et raccordable avec l'autre bout à un réservoir de fluide, où le profil extérieur du ballon (1) en état rempli de fluide est adapté au profil intérieur de la cavité corporelle,
ledit dispositif étant caractérisé en ce que
le ballon (1) est développé en tant que cathéter modelé d'une façon idéale en ce qui concerne son anatomie, le ballon étant développé en forme de coin pour son adaptation au sinus frontal ou à la cavité ethmoïdal de l'homme, et comportant un connecteur en cône (4) qui a pour effet qu'un orifice aux assez grandes dimensions reste ouvert pour l'enlèvement du ballon.

2. Dispositif d'après la revendication 1, caractérisé en ce que le ballon (1) est composé d'un élastomère à silicone élastique.

3. Dispositif d'après la revendication 1 ou 2, caractérisé en ce que le ballon (1) présente un plat (3) qui est conçu d'être essentiellement non prêtant pourtant d'être souple à la flexion.

4. Dispositif d'après la revendication 3, caractérisé en ce que le plat non prêtant se compose d'un élastomère à silicone, dans lequel une insertion est noyée qui se compose d'un tissu en fibres non prêtant.

5. Dispositif d'après la revendication 4, caractérisé en ce que l'insertion consiste en polyester.

6. Dispositif d'après la revendication 4, caractérisé en ce que l'insertion se compose d'un tissu renforcé avec Dacron.

7. Dispositif d'après l'une des revendications 1 à 6, caractérisé en ce que le connecteur (4) va sous l'angle d'environ 60° par rapport au plat non prêtant.

8. Dispositif d'après l'une des revendications 3 à 7, caractérisé en ce que, pour la raison de l'adaptation à la base du crâne ovale par rapport de l'axe longitudinal, le plat non prêtant est développé en tant que surface de support ovale par rapport à son axe longitudinal.

9. Dispositif d'après l'une des revendications 1 à 8, caractérisé en ce que le ballon (1) est joint à un conduit d'amenée mince pour le gonfler et désaérer.

10. Dispositif d'après la revendication 9, caractérisé en ce que le connecteur (4) se compose d'une matière renforcée.
